# EUROPEAN PATENT SPECIFICATION

(11) **EP 1 458 705 B1**
(45) Date of publication and mention of the grant of the patent: **22.07.2009**
(21) Application number: 02805589.5
(22) Date of filing: 13.12.2002
(51) Int. Cl.: C07D 333/22, C07D 333/46

(54) **THIOPHENE DERIVATIVES AND THEIR USE AS FRAGRANTS**
THIOPHENDERIVATE UND IHRE VERWENDUNG ALS RIECHSTOFFE
DERIVES DE THIOPHENE ET LEURS UTILISATIONS EN TANT QU'AROMES ET PARFUMS

(30) Priority: 19.12.2001 US 342150 P; 15.01.2002 US 348580 P; 07.02.2002 US 355052 P; 03.05.2002 US 377914 P; 17.06.2002 US 389298 P; 12.07.2002 WO PCT/US02/22441; 12.07.2002 WO PCT/US02/22120; 20.08.2002 WO PCT/US02/26446; 23.08.2002 US 405653 P
(43) Date of publication of application: 22.09.2004
(73) Proprietor: Flexitral, Inc., Chantilly, VA 20151 (US)
(72) Inventor: TURIN, Luca, London NW1 7NB (GB)
(74) Representative: Bawden, Peter Charles
(86) International application number: PCT/US2002/040018
(87) International publication number: WO 2003/053953

(56) References cited:
- EP-A- 0 219 146
- US-A- 3 549 624
- WEYERSTAHL P ET AL: "Olfactory properties and convenient synthesis of furans and thiophenes related to rose furan and perillene and their isomers" LIEBIGS ANNALEN: ORGANIC AND BIOORGANIC CHEMISTRY, vol. 10, 1 October 1995 (1995-10-01), pages 1849-1853, XP000608611
- DATABASE CROSSFIRE BEILSTEIN [Online] Beilstein Institut zur Förderung der Chemischen Wissenschaften, Frankfurt am Main, DE; XP002233853 & J. CHEM. RES. MINIPRINT, vol. 10, 1995, pages 2364-2379,
- GAGNIANT P ET AL: "Recherche dans la série thiophénique. -I.- Etude de quelques acides omega.2-thiénylaliphatiques de formule générale: C4H3S.(CH2)n.COOH" BULLETIN DE LA SOCIÉTÉ CHIMIQUE DE FRANCE, 1948, pages 1083-1087, XP002233849
- TAMARU Y ET AL: "The palladium catalyzed thienylation of allylic alcohols with 2- and 3-bromothiophenes and their derivatives" TETRAHEDRON, vol. 35, no. 3, 1979, pages 329-340, XP002233850
- ARENA G ET AL: "Thermodynamics of protonation of some five-membered heteroaryl-carboxylates, -alkanoates and -trans-propenoates" JOURNAL OF THE CHEMICAL SOCIETY, PERKIN TRANSACTIONS 2, no. 10, October 1993 (1993-10), pages 1941-1945, XP002233851
- GARRIGUES B ET AL: "Synthèse de 2-tert-butylthiophènes substitués en position 5" BULLETIN DE LA SOCIÉTÉ CHIMIQUE DE FRANCE, vol. 130, no. 1, 1993, pages 58-63, XP002233852
- RABINOWITZ M.H. ET AL: "Design of selective and soluble inhibitors of tumor necrosis factor - alpha converting enzyme (TACE)" JOURNAL OF MEDICINAL CHEMISTRY, vol. 44, no. 24, 22 November 2001 (2001-11-22), pages 4252-4267, XP002266978
- TASHTOUSH H.I. ET AL: "Free-radical coupling of alkyl and aryl halides with electron-deficient alkenes mediated by chromium(II) complexes" CHEMISCHE BERICHTE, vol. 126, no. 7, 2 July 1993 (1993-07-02), pages 1759-1761,

## Description

### FIELD OF THE INVENTION

The present invention relates generally to the field of fragrances. More particularly, the present invention relates to improved muguet/lily-of-the valley (henceforth muguet) derivatives that provide perfumes and other fragrant articles with a fresh, green-floral, odour. These derivatives possess an odour of greater intensity, more desirable character and other useful properties.

### BACKGROUND OF THE INVENTION

Muguet aromachemicals are a major component of many perfumes, and have fresh, green-floral, scent. Many are based on the structure below 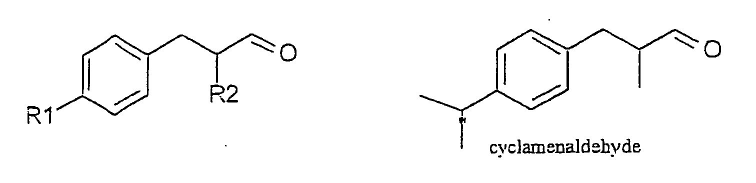 where R1 and R2 can be straight or branched C₁₋₅ alkyl claims. An example of a commonly used material is p-isopropyl alpha-methyl dihydrocinnamaldehyde, also known as cyclamenaldehyde (henceforth, cyclamenaldehyde).

Weyerstahl P et al, "Olfactory properties and convenient synthesis of furans and thiophenes related to rose furan and perillene and their isomers" Liebigs Annalen: Organic and Bioorganic Chemistry, vol. 10, 1 October 1995, pages 1849-1853, describes a process for the synthesis of furans and thiophenes related to Rose Furan and Perillene and their isomers.

Certain cyclic amidines and their non-toxic acid addition salts which are useful as anthelmintic agents are described in US 3,549,624.

The intramolecular oxidative cycloaddition of thiophenes is described in Databased Crossfire Beilstein, Beilstein Institut zur Förderung der Chemischen Wissenschaften, Frankfurt am Main, DE; XP002233853.

Gagniant P et al, "Recherche dans la serie thiophénique. -I.- Etude de quelques acides omega.2-thiénylaliphatiques de formule générale: C4H3S. (CH2)n.COOH" Bulletin de la Société Chimique de France, 1948, pages 1083-1087, describes the synthesis of a series of thiphenes.

The palladium catalysed reaction of allylic alcohols with 2-bromothiophene to provide 3-(2'thienyl) aldehydes or ketones is described in Tamaru et al, "The palladium catalyzed thienylation of allylic alcohols with 2- and 3-bromothiophenes and their derivatives" Tetrahedron, vol. 35, no. 3, 1979, pages 329-340.

Arena G et al, "Thermodynamics of protonation of some five-membered heteroaryl-carboxylates, -alkanoates and -trans-propenoates" Journal of the Chemical Society, Perkin Transactions 2, no. 10, October 1993, pages 1941-1945 reports a study of the thermodynamics of protonation of some five-membered heteroaryl-carboxylate, -alkanoates and -trans-propenoates.

Garrigues B et al, "Synthèse de 2-tert-butylthiphènes substitués en position 5" Bulletin de la Société Chimique de France, vol. 130, no. 1, 1993, pages 58-63, describes the synthesis of 2-tert-butylthiphenes which are substituted at the 5 position.

Thiophene compounds that may be used as inhibitors of tumor necrosis factor -α converting enzyme (TACE) are described in Rabinowitz H et al, "Design of selective and soluble inhibitors of tumour necrosis factor - alpha converting enzyme (TACE)" Jorunal of Medicinal Chemistry, vol. 44, no. 24, 22 November 2001, pages 4252-4267.

A method which may be used to produce thienyl compounds which is mediated by Chromium (II) complexes is described in Tashtoush H I et al, "Free-radical coupling of alkyl and aryl halides with electron-deficient alkenes mediated by chromium (II) complexes "Chemische Berichte, vol. 126, no. 7, 2 July 1993, pages 1759-1761.

It would be desirable to develop muguet derivatives with improved odorant intensity, while maintaining their fresh, floral, muguet character. The present invention provides such derivates.

### SUMMARY OF THE INVENTION

Improved fragrances and flavourings that have an increased odorant intensity relative to cyclamenaldehyde are disclosed. In particular, cyclamenaldehyde derivatives that maintain the flavour and/or fragrance characteristics of cyclamenaldehyde, while increasing the odor intensity relative to cyclamenaldehyde are disclosed. Also disclosed are methods of making the derivatives, and articles of manufacture including the derivatives.

The present invention provides compounds having one of the following formulae 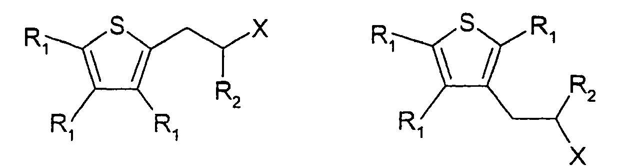 wherein each R₁ and R₂ are, independently, H, C₁₋₅ alkyl or C₁₋₅ substituted alkyl, X -C(=O)H,
wherein the substituents on the substituted alkyl groups are selected from the group consisting of halo, and hydroxy, and wherein at least one R₁ is isopropyl.

The present invention also provides the use as a flavour or fragrance of a compound having one of the following formulae: 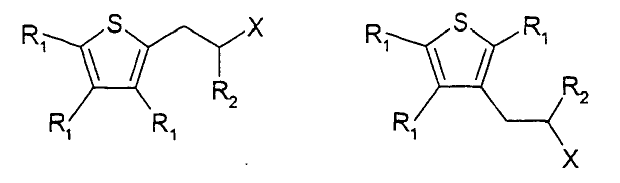 wherein each R₁ is independently, H or C₁₋₅ alkyl or C₁₋₅ substituted alkyl, R₂ is H, C₁₋₅ alkyl or C₁₋₅ substituted alkyl, X is -C(=O)H,
wherein the substituents on the substituted alkyl groups are selected from the group consisting of halo and hydroxy.

The cyclamenaldehyde derivatives may be prepared by replacing the phenyl ring in cyclamenaldehyde with a thiophene ring, which can otherwise be unsubstituted, or additionally substituted at the 2 and/or 3 position with one or two lower alkyl, preferably methyl groups. Although not within the scope of the claims, the aldehyde group in cyclamenaldehyde can further be replaced with an acetal, methyl ether or nitrile functional, group. Acetal groups can provide the compounds with a long lasting flavor or fragrance, where the acetals slowly hydrolyze to provide the parent aldehyde compounds. When the acetal substitution is coupled with the replacement of the phenyl ring with thiophene, which increases the odorant intensity, the cyclamenaldehyde derivatives can provide a similar odorant intensity to cyclamenaldehyde over a relatively longer period of time.

Examples of suitable articles of manufacture include perfumes and colognes, candles, air fresheners, and disinfectant compositions.

### DETAILED DESCRIPTION OF THE INVENTION

Improved cyclamenaldehyde derivatives, which can be used, for example, as fragrances and flavorings that have an increased odorant intensity relative to cyclamenaldehyde, are disclosed. These cyclamenaldehyde derivatives have odor characteristics that are similar to cyclamenaldehyde. Further, in some embodiments, it has been observed that the presence of the cyclamenaldehyde derivatives actually increases the intensity of other odorants. This is apparent in two contexts: first, the presence of even low concentrations (≤ 1%) of the improved derivative gives greater depth, definition and radiance to fragrance compositions of different types. Second, prolonged smelling of even low concentrations of the improved derivative heightens the sense of smell to all ambient olfactory stimuli, e.g. food, fragrances, etc.

### I. Improved cyclamenaldehydes

Formulas 1 and 2 below represent cyclamenaldehyde derivatives that can be modified using the chemistry described herein to replace the phenyl ring in cyclamenaldehyde with a thiophene ring.

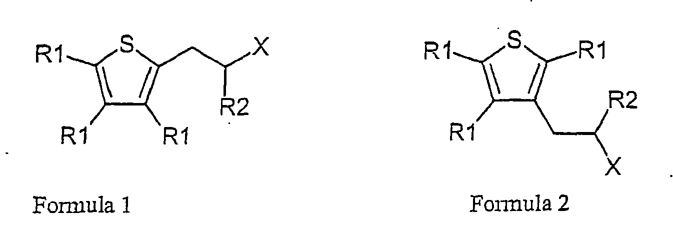

The derivative in which the phenyl ring in cyclamenaldehyde is replaced with a thiophene ring, but no other replacements are made, is shown in the above formula where X is - C(=O)H, and one of R₁ is an isopropyl group and the others are H, and R2= methyl. Thiophene and benzene differ in that benzene permits ortho, meta and para substitution, whereas thiophene rings offer substitution at positions one carbon and two carbons away from the ring sulfur atom. It is believed that the closest chemical analogy to cyclamenaldehyde is obtained where the carbons adjacent to the ring sulfur atom include the isopropyl and aldehyde-containing side-chain present in cyclamenaldehyde. However, suitable odorants are obtained when any of R₁ and R₂ is, independently, H, C₁₋₅ alkyl, C₁₋₅ halo-substituted alkyl, or C₁₋₅ hydroxy-substituted alkyl, and X is -C(=O)H.

The formula below represents a preferred cyclamenaldehyde derivative

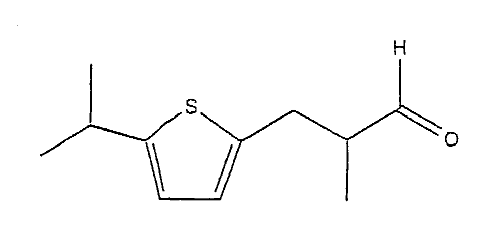

### II Methods for Preparing the Cyclamenaldehyde derivatives

The cyclamenaldehyde derivatives of Formulas 1 and 2 can be prepared using known thiophene chemistry. For example, alkyl, groups can be placed on the thiophene ring using alkyl halides and a suitable Lewis acid catalyst, for example, aluminum chloride. The aldehyde-containing side chain of cyclamenaldehyde can be attached by reacting acrolein with thiophene or an alkylated thiophene using an appropriate Lewis acid catalyst. If other aldehyde-containing side chains are desired, other olefin-containing groups can be used. Functional groups that are sensitive to the presence of Lewis acids can be protected using known protecting groups before performing the alkylation reactions, and deprotected after the alkylation reactions are complete. This can be particularly preferred if any functional groups tend to react with thiophene rings in the presence of a Lewis acid catalyst.

The aldehyde group, if present, can be protected, for example, as an acetal during the alkylation reactions, and deprotected as desired after the reactions take place. In one embodiment, however, the acetals (for example, dimethyl, diethyl, or ethylene glycol ketals) are not deprotected to the aldehyde, such that the flavoring or fragrance includes a portion of or is entirely made up of the acetals. The acetals can then slowly hydrolyze over time, releasing the muguet/lily of the valley odor.

### III. Articles of Manufacture Including the cyclamenaldehyde derivatives

The cyclamenaldehyde derivatives can be included in virtually any article of manufacture that can include cyclamenaldehyde, or for that matter, other fragrances, whether natural or artificial. The cyclamenaldehyde derivatives are particularly well suited for use in both fine and functional perfumery. The cyclamenaldehyde derivatives can be used in applications like soaps, shampoos, body deodorants and antiperspirants, solid or liquid detergents for treating textiles, fabric softeners, detergent compositions and/or all-purpose cleaners for cleaning dishes or various surfaces, for both household and industrial use. Of course, the use of the compounds is not limited to the above-mentioned products, as they be used in other current uses in perfumery, namely the perfuming of soaps and shower gels, hygiene or hair-care products, as well as of body deodorants, air fresheners and cosmetic preparations. These uses are described in more detail below.

### Perfume Compositions

The compounds can be used as perfuming ingredients, as single compounds or as mixture thereof, preferably at a range of at least about 30 % by weight of the perfume composition, more preferably at a range of at least about 60 % by weight of the composition. The compounds can even be used in their pure state or as mixtures, without added components. The olfactory characteristics of the individual compounds are also present in mixtures thereof, and mixtures of these compounds can be used as perfuming ingredients. This may be particularly advantageous where separation and/or purification steps can be avoided by using compound mixtures.

In all cited applications, the cyclamenaldehyde derivatives can be used alone or in admixture with other perfuming ingredients, solvents or adjuvants of current use in the art. The nature and the variety of these co-ingredients do not require a more detailed description here, which, moreover, would not be exhaustive, and the person skilled in the art will be able to choose the latter through its general knowledge and as a function of the nature of the product to be perfumed and of the desired olfactive effect.

The proportions in which the cyclamenaldehyde derivatives can be incorporated in the various products vary within a large range of values. These values depend on the nature of the article or product that one desires to perfume and the odor effect searched for, as well as on the nature of the co-ingredients in a given composition when the compounds are used in admixture with perfuming co-ingredients, solvents or adjuvants of current use in the art.

As an example, the cyclamenaldehyde derivatives are typically present at concentrations between about 0.1 and about 10%, or even more, by weight of these compounds relative to the weight of the perfuming composition in which they are incorporated. Far lower concentrations than those mentioned above can be used when the compounds are directly applied for perfuming the various consumer products cited beforehand.

The compounds can also be used in body deodorants and antiperspirants, for example, those containing aluminum salts. These embodiments are described in more detail below.

### Time Release Formulations

Advantageously, all or a portion of those derivatives that include an aldehyde group can be modified to include an acetal group, which can cause the formulations to release fragrance over a period of time as the acetal hydrolyzes to form the aldehyde compound.

The present invention will be better understood with reference to the following nonlimiting example.

### Example 1: Preparation of 2 & 3 Isopropyl Thiophene

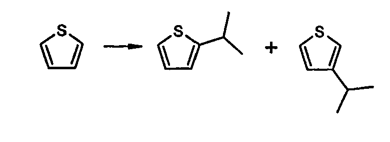

Isopropyl chloride (13mL) was added dropwise to anhydrous aluminum chloride (23.8g) in dry dichloromethane at -78°C. The solution was stirred for 10 minutes following the addition then thiophene (13ml, distilled from KOH) was added (color changed to yellow). The mixture was allowed to reach room temperature as the cold bath (cardice) slowly warmed and then stirred at this temperature for 2 days (color change to red). The mixture was poured onto ice, the organic layer separated and the aqueous layer extracted with dichloromethane (2 x 50mL). The combined organic layers were washed with water, 1% KOH solution and water again before drying over sodium sulfate and evaporating to dryness. Distillation at atmospheric pressure (146°C) afforded 9 mL of the title compounds as a mixture of isomers (7:3, 2-thiophene:3-thiophene).

### Alkylation of 2 & 3 Isopropyl Thiophene

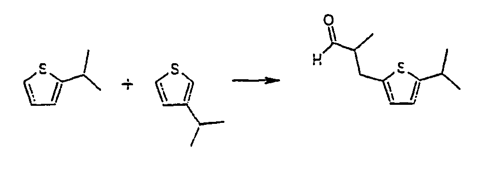

To a stirred mixture of 2-methyl acrolein (5mL), pTSA (20mg) and hydroquinone (20mg) at 0°C was added 5mL of the above compound mixture. The solution was allowed to reach RT over 2 hours then stirred overnight. Flash column chromatography using hexane/dichloromethane (1:1) afforded the structure shown as a colorless oil (1g).

The allylation reaction was not optimized. The recovered starting material contained the same isomer mixture (7:3, 2-thiophene:3-thiophene) as was present at the start of the reaction.

## Claims

1. A compound having one of the following formulae: 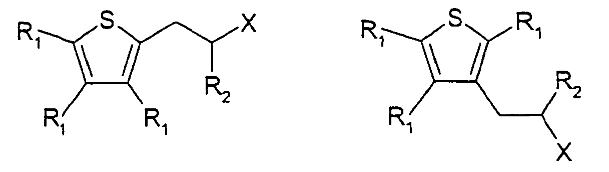 wherein each R₁ and R₂ are, independently, H, C₁₋₅ alkyl or C₁₋₅ substituted alkyl, X -C(=O)H,
wherein the substituents on the substituted alkyl groups are selected from the group consisting of halo, and hydroxy, and wherein at least one R₁ is isopropyl.

2. A compound according to claim 1 having the following formula:

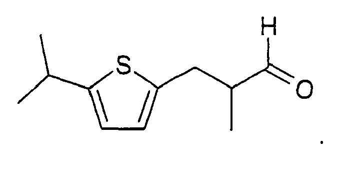

3. A composition comprising a compound according to claim 1 or 2, together with other perfuming ingredients, solvents, or adjuvants of current use in the art of perfumery.

4. A composition according to claim 3, wherein the compound is present in an amount of at least 30 percent by weight.

5. A composition according to claim 3, wherein the compound is present in an amount of at least 60 percent by weight.

6. A perfuming composition or perfumed article containing as a perfuming ingredient a compound or a mixture of compounds according to claim 1 or 3 or a composition according to claim 3.

7. The use as a flavour or fragrance of a compound having one of the following formulae: 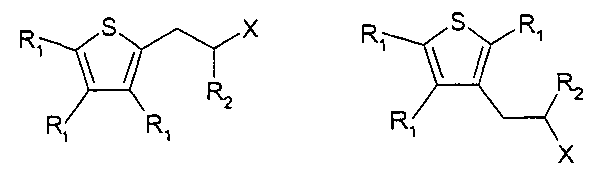 wherein each R₁ is independently, H or C₁₋₅ alkyl, or C₁₋₅ substituted alkyl, R₂ is H, C₁₋₅ alkyl or C₁₋₅ substituted alkyl, X is -C(=O)H,
wherein the substituents on the substituted alkyl groups are selected from the group consisting of halo and hydroxy.

8. A perfumed article in the form of a perfume or cologne, a soap, a bath or shower gel, a shampoo or other hair care product, a cosmetic preparation, a body deodorant or antiperspirant, an air freshner, a fabric detergent or softener or an all-purpose household cleaner and comprising a compound or a mixture of compounds according to claim 1 or 2 or as defined in claim 7.

9. A body deodorant or antiperspirant, containing as a perfuming ingredient a compound or a mixture of compounds according to claim 1 or 2 or as defined in claim 7.

10. A body deodorant or antiperspirant according to claim 9, wherein the compound or mixture of compounds is present in admixture with other perfuming ingredients, solvents, or adjuvants of current use in the art.

11. A method to improve, enhance, or modify the odor of a perfuming composition or a perfumed article comprising adding to said composition or said article an effective amount of a compound or a mixture of compounds according to claim 1 or 2 or as defined in claim 7.

12. A method according to claim 11, wherein the compound or mixture of compounds is present in admixture with other perfuming ingredients, solvents, or adjuvants of current use in the art.

13. A method according to claim 11, wherein the compounds are present in an amount of at least 30 percent by weight.

## Patentansprüche

1. Verbindung mit einer der folgenden Formeln: 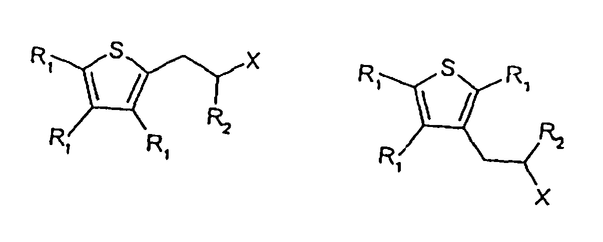 wobei R₁ und R₂ jeweils unabhängig voneinander H₂C₁₋₅ Alkyl oder ein C₁₋₅ substituiertes Alkyl und X -C(=O)H sind,
wobei die Substituenten der substituierten Alkylgruppen aus der Gruppe gewählt sind, die aus Halogen und Hydroxy besteht, und wobei zumindest ein R₁ Isopropyl ist.

2. Verbindung nach Anspruch 1 mit der folgenden Formel :

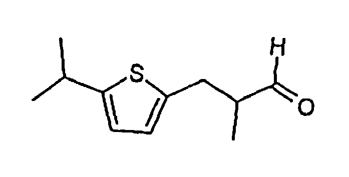

3. Zusammensetzung mit einer Verbindung nach Anspruch 1 oder 2, zusammen mit anderen parfümierenden Inhaltsstoffen, Lösungsmitteln oder Hilfsmitteln die in der Parfümerie zurzeit benutzt werden.

4. Zusammensetzung nach Anspruch 3, bei der die Verbindung in einer Menge von mindestens 30 Gewichtsprozent zugegen ist.

5. Zusammensetzung nach Anspruch 3, bei der die Verbindung in einer Menge von mindestens 60 Gewichtsprozent zugegen ist.

6. Parfümierende Zusammensetzung oder parfümierter Artikel, der als parfümierenden Bestandteil eine Verbindung oder eine Mischung von Verbindungen nach den Ansprüchen 1 oder 3 oder eine Zusammensetzung nach Anspruch 3 enthält.

7. Die Verwendung als Geschmacks- oder Duftstoff einer Verbindung mit einer der folgenden Formeln: 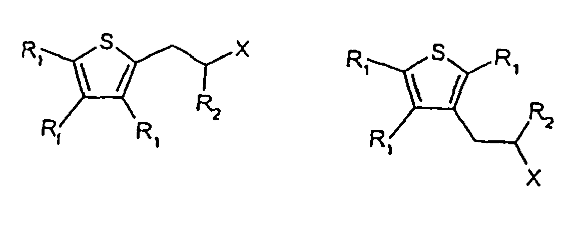 worin R₁ jeweils unabhängig H oder C₁₋₅ Alkyl oder ein C₁₋₅ substituiertes Alkyl ist und R₂ H, C₁₋₅Alkyl oder ein C₁₋₅ substituiertes Alkyl und X -C(=O)H sind,
wobei die Substituenten der substituierten Alkylgruppen aus der Gruppe gewählt sind, die aus Halogen und Hydroxy besteht.

8. Parfümierter Artikel in der Form eines Parfums oder Colognes, einer Seife, eines Bade-oder Duschgels, eines Shampoos oder anderen Haarpflegeprodukts, einer kosmetischen Zubereitung, eines Deodorants oder Antiperspirants für den Körper, eines Lufterfrischers, eines Reinigungsmittels oder Weichmachers für Gewebe oder ein Allzweck-Haushaltsreinigers, welcher eine Verbindung oder eine Mischung von Verbindungen nach den Ansprüchen 1 oder 2 oder nach Anspruch 7 umfasst.

9. Deodorant oder Antiperspirant für den Körper, welche als parfümierenden Bestandteil eine Verbindung oder eine Mischung von Verbindungen nach den Ansprüchen 1 oder 2 oder nach Anspruch 7 enthält.

10. Deodorant oder Antiperspirant für den Körper nach Anspruch 9, bei dem die Verbindung oder Mischung von Verbindungen in Mischung mit anderen parfümierenden Inhaltsstoffen, Lösungsmitteln oder Hilfsmitteln zugegen sind, die zur Zeit im Stand der Technik genutzt werden.

11. Verfahren zur Verbesserung, Förderung oder Modifizierung des Duftes einer parfümierenden Zusammensetzung oder eines parfümierten Artikels, bei welchem der Zusammensetzung oder dem Artikel eine wirksame Menge einer Verbindung oder einer Mischung von Verbindungen nach den Ansprüchen 1 oder 2 oder nach Anspruch 7 hinzugefügt werden.

12. Verfahren nach Anspruch 11, bei dem die Verbindung oder die Mischung der Verbindungen In Mischung mit anderen parfümierenden Inhaltsstoffen, Lösungsmitteln oder Hilfsmitteln zugegen sind, die zurzeit im Stand der Technik genutzt werden.

13. Verfahren nach Anspruch 11, bei dem die Verbindungen in einer Menge von mindestens 30 Gewichtsprozent zugegen sind.

## Revendications

1. Composé ayant l'une des formules suivantes : 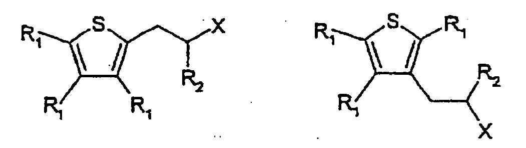 dans lesquelles chaque R₁ et R₂ sont, indépendamment, H, un alkyle en C₁₋₅ ou alkyle en C₁₋₅ substitué, X est -C(=O)H,
dans lesquelles les substituants sur les groupes alkyles substitués sont choisis dans le groupe constitué par un halogène et un hydroxy, et dans lesquelles au moins un R₁ est un isopropyle.

2. Composé selon la revendication 1, ayant la formule suivante :

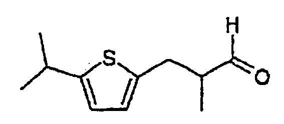

3. Composition comprenant un composé selon la revendication 1 ou 2, conjointement avec d'autres ingrédients parfumants, des solvants ou des adjuvants actuellement utilisés dans la technique de parfumerie.

4. Composition selon la revendication 3, dans laquelle le composé est présent en une quantité d'au moins 30 pour cent en poids.

5. Composition selon la revendication 3, dans laquelle le composé est présent en une quantité d'au moins 60 pour cent en poids.

6. Composition parfumante ou article parfumé contenant en tant qu'ingrédient parfumant un composé ou un mélange de composés selon la revendication 1 ou 3 ou une composition selon la revendication 3.

7. Utilisation, en tant que flaveur ou fragrance, d'un composé ayant l'une des formules suivantes : 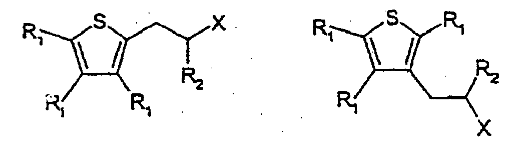 dans lesquelles chaque R₁ est indépendamment H ou un alkyle en C₁₋₅ ou alkyle en C₁₋₅ substitué, R₂ est H, un alkyle en C₁₋₅ ou alkyle en C₁₋₅ substitué, X est -C(=O)H,
dans lesquelles les substituants sur les groupes alkyles substitués sont choisis dans le groupe constitué par un halogène et un hydroxy.

8. Article parfumé, sous la forme d'un parfum ou d'une eau de Cologne, d'un savon, d'un gel pour le bain ou la douche, d'un shampoing ou d'un autre produit pour le soin des cheveux, d'une préparation cosmétique, d'un déodorant ou antisudorifique corporel, d'un assainisseur d'air, d'un détergent ou assouplissant pour le linge ou d'un nettoyant ménager tous usages, et comprenant un composé ou un mélange de composés selon la revendication 1 ou 2 ou tels que définis dans la revendication 7.

9. Déodorant ou antisudorifique corporel, contenant en tant qu'ingrédient parfumant un composé ou un mélange de composés selon la revendication 1 ou 2 ou tels que définis dans la revendication 7.

10. Déodorant ou antisudorifique corporel selon la revendication 9, dans lequel le composé ou le mélange de composés est présent en mélange avec d'autres ingrédients parfumants, des solvants ou des adjuvants actuellement utilisés dans la technique.

11. Procédé pour améliorer, exalter ou modifier l'odeur d'une composition parfumante ou d'un article parfumé, comprenant l'addition à ladite composition ou audit article d'une quantité efficace d'un composé ou d'un mélange de composés selon la revendication 1 ou 2 ou tels que définis dans la revendication 7.

12. Procédé selon la revendication 11, dans lequel le composé ou le mélange de composés est présent en mélange avec d'autres ingrédients parfumants, des solvants ou des adjuvants actuellement utilisés dans la technique.

13. Procédé selon la revendication 11, dans lequel les composés sont présents en une quantité d'au moins 30 pour cent en poids.
